# EUROPEAN PATENT APPLICATION

(11) **EP 4 167 242 A1**
(43) Date of publication of application: **19.04.2023**
(21) Application number: 21207909.9
(22) Date of filing: 12.11.2021
(51) Int. Cl.: G16H 30/40, G16H 50/20

(54) **ULTRASOUND IMAGING IN A DISTRIBUTED SYSTEM**

(30) Priority: 14.10.2021 US 202163255623 P
(71) Applicant: Koninklijke Philips N.V., 5656 AG Eindhoven (NL)
(72) Inventor: BHARAT, Shyam, Eindhoven (NL); SUTTON, Jonathan Thomas, Eindhoven (NL); WILSON, Martha Gail Grewe, Eindhoven (NL)
(74) Representative: Philips Intellectual Property & Standards

(57) **Abstract**

A computer-implemented method is provided for facilitating the acquisition of ultrasound imaging data over an extended period of time, with improved consistency in the image view which is captured. A first set of ultrasound imaging data, comprising one or more images representing one or more views of the anatomy of a patient, is acquired and stored in a datastore. This image data is then used as reference data for subsequent image acquisition, wherein subsequent image data is compared in real time with the reference image data to obtain a measure of a degree of correspondence between the views depicted. This can be used to guide the acquisition of the new image data so that it is consistent with the previous image data. For example, when the view represented in the newly acquired image data sufficiently matches that in the reference data, an alert message can be issued to the user.

## Description

### FIELD OF THE INVENTION

The present invention relates to a method for ultrasound imaging in a distributed system, in particular for aiding longitudinal ultrasound imaging of a same patient.

### BACKGROUND OF THE INVENTION

Ultrasound imaging can be used in a variety of clinical settings to evaluate patients (e.g. in an intensive care setting, in an operating room and, in a general ward).

Ultrasound image acquisition may be performed for a patient at multiple time points, either over a single short-term monitoring period (e.g. during surgery), or over a long term period. In such contexts, acquisition of consistent image views of an anatomy of interest is important, in order to allow for reliable clinical comparison over time.

By way of illustration, two example modes of ultrasound imaging which are used to ultrasonically evaluate patients are Transthoracic echocardiography (TTE) and transesophageal echocardiography (TEE). TTE is non-invasive and therefore has a wider range of use than TEE. It can be used outside of emergency settings. It also carries a lower clinical risk to the patient (compared to TEE) and can be performed by operators with lower skill levels due to its non-invasive nature. However, it can be more difficult to acquire the correct views of a given anatomy using TTE than TEE, since the operator needs to place the probe in exactly the correct position on the chest (compared with TEE where the esophagus largely constrains the probe placement).

Ultrasound imaging can be used to monitor cardiac function during surgery. For example, the use of TEE to monitor cardiac function is increasing in popularity in clinical settings, particularly in cases where other modes such as TTE is difficult to perform due to patient habitus, external chest injuries, other external medical equipment, or patient dressings. For example, to view the left ventricle, a TEE probe may be navigated to the mid-esophageal position. If a clinician requires a view of the heart at any time during the surgery, the probe is manually manipulated so as to acquire the desired view.

The use ultrasound for continual monitoring of the heart (e.g. in an intensive care environment or during surgery) has been proposed within the medical community, and is a current area of technical development. Ultrasound imaging in this context is useful not only for acquiring and viewing imagery of the patient anatomy, e.g. the dynamic motion of the heart, but also for obtaining quantitative measurements of physiological parameters, e.g. hemodynamic parameters such as left ventricular (LV) volume, stroke volume (SV), cardiac output (CO), LV Ejection Fraction (LVEF). A trend pattern of these parameters can be tracked over time and used to inform patient treatment.

It is known for example to apply model-based segmentation to acquired ultrasound imagery in order to obtain quantitative measurements. For example, automatic segmentation of one or more chambers of the heart can be performed. Measurements of hemodynamic parameters can be obtained through applying model-based segmentation on an end-diastolic (ED) ultrasound image frame to detect heart chamber boundaries, followed by 2D or 3D tracking of the segmentation boundaries over the other frames of the cardiac cycle.

For 2D imaging, geometric assumptions can be made to extrapolate from acquired single-plane or bi-plane information, to generate measurement information for 3D objects or features, e.g. heart chamber volumes. Example algorithms include modified Simpson's rule, ellipsoidal model using single-plane, bi-plane or unidimensional data, or the hemisphere-cylinder model based on bi-plane data.

For further details on these segmentation methods, reference is made to: Kosaraju A, Makaryus AN. Left Ventricular Ejection Fraction. In: StatPearls [Internet]. Treasure Island (FL): StatPearls Publishing; 2019 Jan-. Available from: https://www.ncbi.nlm.nih.gov/books/NBK459131/.

Longitudinal ultrasound monitoring of a patient over a continuous time period (e.g. over hours) can be challenging because the probe position can drift over time. In an intensive care environment, measurements might be taken by multiple clinicians or specialists at different points in time. Measurements may also be desired at times when a cardiologist, intensivist or other specialist is absent. Furthermore, TTE imaging, by way of example, also presents its own challenges since the same correct view has to be obtained on each occasion from scratch.

Additionally, when generating longitudinal trends for a given measurement (e.g. SV in the case of cardiac imaging), it is important to ensure consistency in the image data provided to the measurement tool. If the image data provided varies in the particular view depicted for example, measurement errors associated with the inconsistent image acquisition can occur, and thus can interfere with the interpretation of longitudinal trends. This may result in inappropriate additions or changes to the medical interventions provided to the patient (e.g., fluid status change, vasopressors, inotropes). This is especially true in 2D imaging approaches, where the possibility of foreshortening (in which the acquired ultrasound plane does not cut through the true apex of the left ventricle) is a significant challenge.

It would be advantageous to provide a solution which enables acquisition of more consistent image data across time.

### SUMMARY OF THE INVENTION

The invention is defined by the claims.

According to examples in accordance with an aspect of the invention, there is provided a computer implemented method, for performance with a distributed computing system. The method comprises:
acquiring at a first ultrasound imaging device first ultrasound imaging data of a subject, including at least one image depicting at least one view of an anatomy of the subject;
storing the first ultrasound imaging data as reference ultrasound image data in a datastore, tagged with subject identifier information;
retrieving from the datastore, at a second ultrasound imaging device, the reference ultrasound image data based on querying the datastore with patient identifier information, wherein the second imaging device is the same as or different to the first imaging device,
acquiring live ultrasound image data at the second ultrasound imaging device;
recurrently generating a live similarity measure indicative of a similarity between the view represented in the reference image data and a view represented in the live ultrasound image data;
generating a user output alert when the similarity measure matches at least one pre-defined criterion,
capturing and storing one or more frames of the live ultrasound image data, tagged with the patient identifier information.

The method thus provides a means for assisting in maintaining consistent ultrasound imaging acquisition views across time, even if being acquired by different operators, with differing skill levels. This object is achieved through storage of a first set of image views, which are then accessible at a later time by another user, and used as a reference for acquisition of subsequent image data of the same patient. Consistency in the views acquired between the two time points is ensured through active comparison of the reference image data with newly acquired live image data, and the generation of a user guidance alert based thereon. Thus, the operator who acquires the second image data is guided to acquire image data which matches the first in terms of its captured view. This concept can be replicated for any number of image acquisition events or periods throughout a patient stay in a medical institution, or even across multiple stays at multiple institutions or care groups. Each time ultrasound image data is to be acquired, at any of a plurality of ultrasound image devices that may be comprised by the overall system, previous reference image data might be retrieved from a centrally accessible data source, and used as a reference guide for acquisition of new image data which corresponds in terms of the view captured to the previous data.

By "view" is meant the particular observation angle and field of view (window of acquisition) of an image relative to an imaged anatomy. For particular anatomies, there are typically a set of standard imaging views which are acquired. For instance, for cardiac imaging, these may include parasternal long-axis and short-axis views, the apical 4-chamber view, and the subxiphoid view. In general, an imaging view will correspond to a particular positioning of the probe relative to the subject, and relative to the anatomy being imaged. For instance, in cardiac imaging, the parasternal views are obtained with the probe positioned just left of the sternum at intercostal space 3 or 4. The apical 4-chamber view of the heart is obtained by placing the probe just below the nipple line at the point of maximal impulse of the heart. The subxiphoid 4-chamber view is obtained with the probe aimed up toward the left shoulder from a position just below the subxiphoid tip of the sternum.

By way of example, the similarity measure could be determined by processing each of the first ultrasound image data and the live ultrasound image data with image segmentation to detect anatomical structures within the image field of each set of image data. For example, a relevant image from the first ultrasound image data is selected, for example it is selected by the first operator in advance. This image is processed with image segmentation. A live image frame from the live ultrasound image data may also be processed with image segmentation. The similarity measure could be derived by comparing the segmentation results for the first image and the live image frame. For instance, by assessing the degree of spatial overlap of one or more anatomical structure (e.g. template matching or object matching), or by comparing binary presence of absence of one or more anatomical structures in each of the first image and live image frame.

In some examples, the method may comprise continuously or recurrently generating a similarity measure and capturing an image frame when the similarity measure exceeds a pre-defined threshold.

When generating the user alert based on the similarity measure, to guide image acquisition, the aforementioned pre-defined criterion may include a pre-defined threshold for the similarity measure.

In some embodiments, the datastore which holds the reference image data may be comprised by a patient monitoring subsystem. Thus, the reference image data is retrieved from a datastore comprised by a patient monitoring subsystem. The patient monitoring subsystem may also receive additional patient measurement data, including vital signs data.

In some examples, at least one of the live ultrasound imaging data and the reference ultrasound image data may be acquired using a transesophageal echocardiography (TEE) ultrasound probe or a transthoracic echocardiography (TTE) probe.

In some embodiments, the method may further comprise capturing and storing at least one frame of the live image data only when the similarity measure meets said pre-defined criterion. In other words, capture of new, second image data is constrained only to capture of image views which sufficiently conform to that represented in the reference image.

In some embodiments, the method may comprise capturing and storing said at least one frame of the live image data: only when the similarity measure meets said pre-defined criterion; and responsive to a capture command from a user input device. In other words, a user actively controls when an image frame is captured for storage, but the time periods at which the user is able to do this are constrained to time periods at which the real-time generated similarity measure meets the pre-defined threshold. Thus a certain level of view conformity is enforced by the system.

In some embodiments, the datastore may store a set of reference images depicting different respective views of the anatomy, each tagged in accordance with the view depicted. In some more particular examples, the datastore may further store a view recommendation indicative of a recommended next view of the anatomy to capture, and wherein the retrieved reference image is one of the set of images depicting the recommended next view.

In some embodiments, the method may further comprise generating probe positioning guidance based on the similarity metric, the guidance for guiding movement of the probe by a user for improving the similarity metric, and outputting the guidance information to a user interface device. Thus, in this case, the user is not just alerted when the view has sufficient correspondence with the reference image view, but is also provide active guidance for moving the positioning of the probe relative to the patient to move closer to the target image view.

In some embodiments, the method may further comprise receiving at the second ultrasound imaging device annotation data for storage in the datastore with the reference image data.

In some embodiments, the method may comprise processing the captured at least one frame of the live image data with a measurement algorithm, to derive one or more anatomical and/or physiological measurements. For example, in the case of imaging of the heart, one or more hemodynamic parameters might be generated. Additionally or alternatively, one or more measurement algorithms might be applied to the first ultrasound imaging data. Acquired measurement data in either case may be stored in the datastore along with the image data for the patient.

A further aspect of the invention provides a processing arrangement comprising: an input/output; and one or more processors. The one or more processors are adapted to perform the following:
retrieve at the input/output from a datastore, reference ultrasound image data depicting at least one view of an anatomy of a subject based on querying the datastore with patient identifier information;
receive at the input/output live ultrasound image data;
recurrently generate a live similarity measure indicative of similarity between the view represented in the reference image data and a view represented in the live ultrasound image data;
generate a user output alert when the similarity measure matches at least one pre-defined criterion; and
capture and store one or more frames of the live image data, tagged with the patient identifier information.

A control signal might be provided to the input/output for controlling a user interface to generate the user output alert.

Another aspect of the invention provides an ultrasound imaging apparatus comprising: a processing arrangement in accordance with any example or embodiment outlined in this disclosure, or in accordance with any claim of this application; and an ultrasound imaging probe for acquiring the live ultrasound imaging data.

Another aspect of the invention provides a system, comprising: an ultrasound imaging apparatus in accordance with any example or embodiment described in this disclosure, or in accordance with any claim of this application; and a datastore for storing reference ultrasound image data.

Another aspect of the invention provides a computer program product comprising code means configured for execution by a processor, wherein, for a processor that is communicatively linked with an ultrasound imaging apparatus; and communicatively linked with a datastore, the code causes the processor to:
receive from the datastore, reference ultrasound image data depicting at least one view of an anatomy of a subject based on querying the datastore with patient identifier information;
receive from the ultrasound imaging apparatus live ultrasound image data;
recurrently generate a live similarity measure indicative of similarity between the view represented in the reference image data and a view represented in the live ultrasound image data;
generate a user output alert when the similarity measure matches at least one pre-defined criterion; and
capture and store one or more frames of the live image data, tagged with the patient identifier information.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 schematically outlines an example system and data flow according to one or more embodiments;
Fig. 2 schematically illustrates in more detail an example system and data flow in accordance with one or more embodiments; and
Fig. 3 illustrates a display of any example patient monitor device which may be comprised by the system according to certain embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a computer-implemented method for facilitating the acquisition of ultrasound imaging data over an extended period of time having consistency in the image view which is captured. A first set of ultrasound imaging data, comprising one or more images representing one or more views of the anatomy of a patient, is acquired and stored in a datastore. This image data is then used as reference data for subsequent image acquisition, wherein subsequent image data is compared in real time with the reference image data to obtain a measure of a degree of correspondence between the views depicted. This can be used to guide the acquisition of the new image data so that it is consistent with the previous image data. For example, when the view represented in the newly acquired image data sufficiently matches that in the reference data, an alert message can be issued to the user.

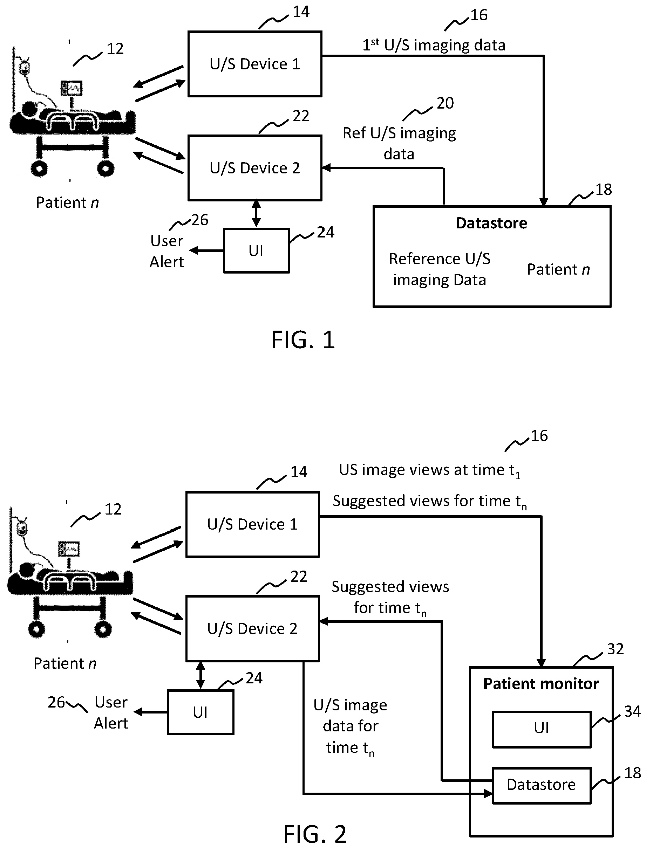

Fig. 1 schematically outlines components of an example system in accordance with an aspect of the invention.

The system comprises one or more ultrasound imaging devices 14, 22. In this example, two ultrasound imaging devices 14, 22 are shown. However, more than two, or only one, is an option in other embodiments. The system further includes a datastore 18 for storing ultrasound imaging data for multiple patients.

The system may be a distributed computing system, meaning that the different components or nodes of the system may be physically isolated from another, but connected in a functional network, for collaborative data acquisition, analysis, storage and user presentation.

The system is for implementing a computer implemented method, for aiding the acquisition of ultrasound imaging data over an extended period of time, which has consistency in the ultrasound image views which are obtained. This computer implemented method forms a further aspect of the present invention. The steps of an example computer-implemented method in accordance with one or more embodiments of the invention will now be outlined in more detail, with reference to components of the system of Fig. 1.

The example method comprises a step of acquiring at a first ultrasound imaging device 14 ultrasound imaging data 16 of a subject 12, including at least one image depicting at least one view of an anatomy of the subject.

The method further comprises storing the first ultrasound imaging data 16 as reference image data in a datastore 18, tagged with subject identifier information. The datastore, in some embodiments, may be a datastore comprised by a patient monitoring subsystem, so that the patient monitoring subsystem acts as a central hub of the system. The patient monitoring system might at the same time be arranged to receive biological sensor data relating to the patient 12, and adapted to display this on a display of a user interface. The display of the user interface may also be controlled to display the acquired first ultrasound imaging data. In some examples, the patient monitoring subsystem, or a separate processing component connected to the patient monitoring subsystem, may be adapted to process the ultrasound imaging data and obtain from it one or more quantitative anatomical and/or physiological measurements, e.g. hemodynamic measurements. The datastore may instead be located elsewhere. It may be a separate dedicated data storage node, e.g. a storage server, e.g. at a central location in the medical institution. It may be a cloud-based data storage means, accessible via an internet connection.

The method further comprises, retrieving from the datastore 18, at a second ultrasound imaging device 22, the reference ultrasound image data based on querying the datastore with patient identifier information. For example an operator at the second ultrasound imaging device 22 inputs the patient identifier information to the second ultrasound imaging device 22 in a setup or initiation step, and this prompts retrieval of the relevant ultrasound image data from the datastore 18. The second imaging device 22 may in fact be the same imaging device as the first (i.e. there is only one imaging device), but being operated at a later time, for example by a different operator to the one who acquired the first ultrasound imaging data 16. Alternatively, the second ultrasound imaging device 22 may be a different imaging device, which may be located at the same physical location, e.g. wheeled in at a later time to the same patient bed, or could be at a different physical location, e.g. the patient has been moved to a different ward or care unit and imaging is recommenced at the new location.

The method further comprises acquiring live ultrasound image data of the patient anatomy at the second ultrasound imaging device 22.

The method further comprises recurrently generating a live similarity measure indicative of a similarity between the view represented in the reference image data and a view represented in the live ultrasound image data. In other words, as the operator of the second ultrasound imaging device is acquiring imagery of the patient, a similarity measure is concurrently calculated in the background, indicative of the degree to which the view that is being captured matches that of the reference ultrasound data acquired by the operator of the first ultrasound imaging device 14. This has the aim of maximizing consistency in the views of the ultrasound image data acquired at the two different time points.

The method may further comprise generating a user output alert when the similarity measure matches at least one pre-defined criterion, e.g. the similarity meets or exceeds a minimum similarity threshold. This indicates to a user that they have found the correct view and can stop adjusting the probe position. In other examples, the similarity measure might simply be communicated to the user through a user interface device, e.g. displayed on a display device of the ultrasound imaging device 22.

The method further comprises capturing and storing one or more frames of the live ultrasound image data, tagged with the patient identifier information. This may be stored in the datastore 18, associated with the patient identifier information.

Computing of the similarity measure may comprise application of an image matching operation, wherein a reference image from the reference image data and the at least one frame from the live image data are compared through a process in which one image is matched against the other. A metric indicative of a degree to which the images match is the output. Object detection is one good way to perform image matching in this way. Image template matching is another good way to perform image matching. Various methods for image comparison and matching are known in the art, and will be apparent to the skilled person, which include object detection and cross correlation for instance.

One example of a suitable template matching using a cross correlation approach is outlined in the paper: Briechle, K and Hanebeck, U. D., "Template matching using fast normalized cross correlation", Proc. SPIE 4387, Optical Pattern Recognition XII, (20 March 2001).

Another example using template matching is outlined in the following Online article: https://www.pyimagesearch.com/2021/03/22/opencv-template-matching-cv2-matchtemplate/, a copy of which was retrieved on 28 September 2021.

To further illustrate the concept of the invention, an example system and computer-implemented method in accordance with at least one set of embodiments will now be outlined in detail, with reference to Fig. 2.

In this example, the system includes a patient monitor 32, and wherein the datastore 18 previously mentioned is comprised by the patient monitor 32. The patient monitor also includes a user interface 34.

The system permits bi-directional communication and information flow between each of one or more ultrasound imaging apparatuses 14, 22 comprised by the system, and the patient monitor. This facilitates imaging, analysis, and patient monitoring workflows involving multiple users (for example including clinical experts and non-experts) and potentially multiple ultrasound platforms (e.g. cart-based ultrasound imaging console, mobile ultrasound imaging probe, or smartphone or tablet computer based devices). In other words, a plurality of different types and classes of node device can connect to the distributed computing system and contribute to a common patient workflow. The system of this set of embodiments enables a multi-stage workflow described below. Although, for illustrative purposes, the system is described with reference to cardiac imaging in particular, and with reference to TTE and TEE probe imaging even more particularly, the concepts are equally applicable to the imaging of other anatomies, with other types of imaging device. In general, the principles of the computer-implemented method which is performed are most advantageously applicable for cases where longitudinal monitoring is needed (e.g., lung monitoring, or monitoring of any other anatomical structure). Longitudinal means over an extended period which may be a continuous extended period, e.g. hours of monitoring, or an extended period with one or more interruptions, e.g. where the patient is moved between different care units etc. It in general means monitoring over a period longer than a single ultrasound examination event.

The data flow of the method according to the embodiment of Fig. 2 may, in more detail, be as follows. The data flow can be understood as comprising two phases. A first phase comprises initial patient evaluation by a first (e.g. expert) user of a first ultrasound imaging apparatus. The second phase comprises a subsequent patient evaluation by a second user, who may be anon-expert user of the ultrasound imaging apparatus and/or not as clinically skilled as the first user.

With reference to Fig. 2, in the first phase, the workflow and data flow may be as follows.

When the patient 12 gets an ultrasound evaluation for the first time (at time ti), it is assumed to be by an expert user skilled in the use of ultrasound. The expert user evaluates the patient using the first ultrasound imaging device 14 (e.g. TTE or TEE imaging). The user acquires all of the views known to be clinically relevant and required. The user may select views that yield the most clinically useful images for that patient, and either mark or tag these images, or delete the other images which are not part of the subset selected as most clinically useful. The image data may in one or more other ways be annotated, edited or pre-processed by the first expert user. The user may label one or more images or subsets of images according to a view which they represent. In other words, the user may curate the images. The user may generate one or more quantitative measurements from the images, manually or using automated algorithms as discussed previously in this disclosure.

The curated images and/or measurements acquired by the first user at the first ultrasound imaging device 14 are transferred to the patient monitor subsystem 32, where they are stored in a datastore 18 comprised by the patient monitor subsystem. The patient monitor subsystem may include one or more client patient monitor devices, which may include one or more bedside patient monitor devices, and may include a central patient monitor console to which each client device is connected. The central monitoring console may house the datastore. The first image data 16 may be displayed on a display of a user interface 34 of the patient monitor subsystem. The patient monitoring system optionally may at the same time be arranged to receive biological sensor data relating to the patient 12, and adapted to display this on a display of a user interface.

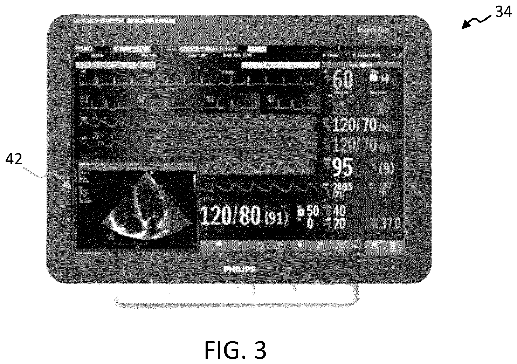

The display of the user interface may also be controlled to display the acquired first ultrasound imaging data. An example user interface 34 of the patient monitoring subsystem is shown in Fig. 3 for illustration. Here, the first ultrasound image data is displayed in window 42 of the display, and other physiological parameter data (e.g. vital signs) are shown in other areas of the display, including trend data.

In a variation, instead of the image data 16 being transferred to the patient monitor subsystem for storage, a bridge processing module may be provided (not shown), which is connected to a patient monitor subsystem 32, and wherein the bridge processing module comprises the datastore 18. For example, the bridge processing module may be communicatively accessible to both the patient monitor subsystem and each of the one or more ultrasound imaging devices 14, 22 which are comprised by the system.

In addition to acquiring the image data, the first user may also input at the first imaging device one or more imaging recommendations, each indicative of one or more recommended views or images to be acquired at a specified subsequent time point. The recommendation may be generic in terms of its timing, i.e. a recommendation simply for a next imaging acquisition event. Alternatively, the timing may be specific, e.g. recommended view(s) for each of one or more specific future time points, tₙ. This recommendation might for instance be based on the expert user's experience during this first acquisition with respect to the best available views, acoustic windows, specifics of the patient anatomy and so on.

The generated recommendations may be transferred to the datastore 18 along with the acquired first image data, for storage in a data record that is associated with the patient. For example, the image data and recommendation(s) may each be tagged with patient identifier information so that it can later be accessed by querying the database with the patient identifier information.

The second phase is a subsequent ultrasound examination by a further user, who may be a non-expert user, not very familiar with ultrasound.

At a subsequent time point, when ultrasound imaging is deemed necessary for the patient, a second user of the system can use any second ultrasound device 22 connected to the system (of which there may be multiple, in different physical locations) to image the patient. The ultrasound imaging device 22 used to image the patient on the second occasion may be the same or different to the ultrasound imaging device 14 used to acquire the first image data.

By way of example, the user at the second imaging device 22 could input patient identifier information to initiate the device for use with the patient. The ultrasound device then, either automatically, or upon prompt by a user through input at a user interface, communicates with the datastore 18 to retrieve stored image reference image data for the patient. The datastore is for example queried using the patient identifier information. In the present case, the first image data 16 that was stored in the datastore 18 by the expert user is found and is retrieved. All of the image data in the datastore 18 may be retrieved or just a subset of it. For example, the user may indicate the particular examination or monitoring protocol that they plan to implement, and the most appropriate images therefor may be retrieved. The retrieved images are transferred from the datastore 18 to the second ultrasound imaging device 22.

Where the first user also provided view recommendations, these may also be transferred to the second ultrasound imaging device 22. In some examples, these may be communicated to the second user by means of a user interface 24 of the ultrasound imaging device. In some examples, the view recommendations may be utilized to determine which of potentially a plurality of different image views stored in the datastore 18 associated with the patient are transferred to the second ultrasound imaging device.

For example, the datastore 18 may store a set of reference images depicting different respective views of the anatomy, each tagged in accordance with the view depicted, and wherein the datastore further stores the aforementioned view recommendation indicative of a recommended next view of the anatomy to capture, and wherein the reference image retrieved and sent to the second ultrasound imaging device 22 is one of the set of images depicting the recommended next view.

The second ultrasound imaging device 22 then uses the ultrasound image data retrieved from the datastore 18 as reference ultrasound image data for use in guiding acquisition of a second set of ultrasound data. For example, the ultrasound imaging device 22 may provide, via the user interface, acquisition assistance to the second user to match the current acquisition to the reference image(s).

In some examples, the acquisition assistance can be in the form of view recognition, wherein live ultrasound image data being acquired by the second user is analyzed in real time, with reference to the reference image data, to determine a live similarity measure between the view represented in the reference image data and a view represented in the live ultrasound image data. The user interface 24 of the second ultrasound imaging device 22 may be controlled to generate a user output alert when the similarity measure matches at least one pre-defined criterion, for example, when a certain threshold has been met for the similarity measure.

Additionally or alternatively the acquisition assistance can be in the form of active probe guidance, wherein a processor of the ultrasound imaging device generates user guidance instructions for guiding movement of the probe so as to improve a similarity measure between the view in the live image data and a view represented in the reference image.

The method further comprises capturing one or more frames of the live ultrasound image data and storing the imaging data in the datastore, for example tagged with the patient identifier information, and tagged with the acquisition time of the data. In some cases, the ultrasound image data may be captured from the second imaging device 22 only when the similarity measure meets said pre-defined criterion. In some cases, the method comprises capturing and storing said at least one frame of the live image data only when the similarity measure meets said pre-defined criterion, and only responsive to a capture command input by a user from a user input device 24. In other words, the user controls the exact timing of image data capture, but wherein the possible timings are constrained according to the similarity measure between a live view of the imaging device and a view of the reference imaging data meeting the pre-defined criterion.

Each of the first 14 and second 22 ultrasound imaging devices, and the patient monitor subsystem, mentioned above comprise a processing arrangement comprising one or more processors which may implement one or more of the steps of the method outlined above.

In some cases, the method may further comprise processing the captured at least one frame of the live image data with an anatomical measurement algorithm, to obtain one or more anatomical or physiological measurements. This processing may be done at the ultrasound imaging device 22 itself, or may be done elsewhere, for example at the patient monitor subsystem 32.

To summarize, the workflow of the embodiment of Fig. 2 may be outlined as follows. First ultrasound image data 16 for a given patient, n, is acquired at one ultrasound imaging device of the system, e.g. by an expert user, at a first time *t₁.* The user may annotate and curate the images. Measurements may be generated from the images. The curated images and/or measurements from the initial expert user acquisition are transferred to the patient monitor 32, preferably along with a recommendation for the desired views and/or images to be acquired for the same patient, n, at a next time point, or at a specified future time point, *tₙ*. At a subsequent time point, for instance when ultrasound imaging is deemed necessary for the patient, or in accordance with a timing specified in the recommendation, the system retrieves the first images 16 for the patient from a database in the patient monitor datastore 18 and transfers them to the current ultrasound system being used as reference images. The system then uses the transferred image(s) as the reference image(s) and provides acquisition assistance to the user with respect to the reference image(s).

In accordance with any of the examples or embodiments of the system and computer-implemented method outlined above, there may be further components or devices comprised by the system. For example, the system may further comprise one or more mobile computing devices (e.g. smartphones or tablet computers) which are communicatively connected, linked or networked with the other components of the system.

In accordance with any of the examples or embodiments of the system and computer-implemented method outlined above, there are various options with regards to the data flow between components of the system. In general, it is to be understood that the method is for implementation by a distributed computing system, and that therefore functions or method steps recited as being implemented by one particular component may in fact, in other embodiments, be implemented by a different component.

For example, in the above-described embodiment, it was noted that annotation data (e.g. notes, labels, tags) may be input by the first user at the first ultrasound imaging device 14, and be sent for storage at the datastore 18 along with the first ultrasound imaging data 16. However, additionally or alternatively, in further embodiments, annotation data associated with the first image data 16 may instead be input by a first user, and/or by a further one or more users, at a different one or more devices or nodes of the system and sent to the datastore 18 for storage. For example, this information could be input at a user interface 34 of the patient monitor subsystem 32 instead of at the first ultrasound imaging device 14. For instance, the patient monitor subsystem may include a central console with a user interface, and wherein the annotation or measurement information may be input at the central patient monitor console device. Additionally or alternatively, the patient monitor may include one or more peripheral (bed-side) monitoring devices and the annotation and/or measurement information may input at a user interface of one or more of these peripheral devices. In some embodiments, the system may further comprise one or more mobile computing devices (e.g. smartphone or tablet computer) communicatively coupled or linked with the datastore 18, and wherein annotation and/or measurement data is input by one or more users at one or more mobile computing devices for transfer to the datastore 18, wherein these one or more users may or may not include the first user (e.g. a different expert user provides the annotations).

Furthermore, the annotation or measurement information may be input at the same time or a different time to the image data acquisition, e.g. later on a mobile device of the first user or different user, and transferred to the datastore for storage.

Furthermore, according to one or more embodiments, the first user acquiring the first ultrasound image data may input at the first ultrasound imaging device an indicted selection of the acquired first ultrasound images which should subsequently be used as a reference image for a subsequent image data acquisition, and this information transferred to the datastore 18 for storage. This information may be input by the first user at the first ultrasound imaging device 14, or may be input (by the same first user or a different one or more users) at a further one or more devices or nodes of the system.

Furthermore, the system may include functionality to permit the first user who acquires the first ultrasound image data 16, and/or another user of the system, to input a recommendation for a time at which subsequent ultrasound image data should be acquired.. For instance, and by way of illustration, an expert clinician reviewing a patient in person may review existing ultrasound images for the patient for instance using a user interface 34 of a patient monitor subsystem 32, and these being retrieved from the datastore 18, and may decide that another ultrasound acquisition would be clinically valuable, acquired in two hours' time. The patient monitor device (or another device of the system, such as a mobile computing device) may permit input by the user at a user interface of an instruction signal by which a future ultrasound imaging event is scheduled, ordered, or recommended, including a specific time for ultrasound imaging acquisition. Optionally, a message might be generated and pushed to one or more further devices of the system, to alter the user(s) of the one or more further devices that the new ultrasound image acquisition has been ordered. Any user (expert or non-expert) can then acquire the recommended subsequent ultrasound imaging data at the specified time, using the guidance functionality which has been described above.

The above description represents just one example set of embodiments, not all features of which are essential to the inventive concept.

For example, instead of using the patient monitor to store the database of patient image data, annotation data, measurement data and/or further data, this data might be stored on a different module such as an interface module (having a datastore comprised thereby) connected between one or more of the ultrasound imaging devices and the patient monitor device.

In general, the various devices and components comprised by the system may be spatially separated from one another, for example distributed at different locations across a medical institution. They may be communicatively connected, linked or networked with one another via one or more network connections. Their communication with one another may be facilitated by one or more components acting as network hubs or servers. Communication between them may be facilitated though a local or wide area network for example, and/or by use of an internet connection. For example, each device might be operable to connect, through a login facility, to a common internet web portal, via which interconnection between the device is facilitated.

Furthermore, it is noted that although 'first' image data and a 'first' user are referred to above, this for convenience of reference and concision only. It is not essential that the ultrasound data referred to as 'first' ultrasound data is in fact the very first ultrasound data acquired for the given patient *n.* The descriptions above intend to merely reflect the concept that one set of image data can be acquired at any of a potential plurality of ultrasound imaging devices of the system, and that one or more images from this data can then be used as a reference for use in acquiring subsequent image data for the same patient at another or the same of the potential plurality of ultrasound imaging devices.

Various embodiments outlined above refer to generating processing ultrasound image data with an anatomical measurement algorithm to derive one or more anatomical or physiological measurements, e.g. hemodynamic measurements. For example, one or more embodiments may comprise processing the captured at least one frame of the live image data with an anatomical measurement algorithm. In one or more embodiments, the first images captured at the first ultrasound imaging device 14 may be processed with an anatomical measurement algorithm to derive one or more anatomical or physiological measurements.

By way of example, the measurement algorithm may employ use of model based segmentation (MBS) to segment boundaries of one or more anatomical structures and to thereby derive dimensional measurements and/or functional measurements such as movement patterns or fluid flow measurements.

For example, for cardiac imaging ultrasound imaging can be used for obtaining quantitative measurements of hemodynamic parameters such as left ventricular (LV) volume, stroke volume (SV), cardiac output (CO), LV Ejection Fraction (LVEF). A trend pattern of these parameters can be tracked over time and used to inform patient treatment.

It is known for example to apply model-based segmentation to acquired ultrasound imagery in order to obtain quantitative measurements For example, automatic segmentation of one or more chambers of the heart can be performed. Measurements of hemodynamic parameters can be obtained through applying model based segmentation on an end-diastolic (ED) ultrasound image frame to detect heart chamber boundaries, followed by 2D or 3D tracking of the segmentation boundaries over the other frames of the cardiac cycle.

For 2D imaging, geometric assumptions can be made to extrapolate from acquired single-plane or bi-plane information, to generate measurement information for 3D objects or features, e.g. heart chamber volume. Example algorithms include modified Simpson's rule, ellipsoidal model using single-plane, bi-plane or unidimensional data, or the hemisphere-cylinder model based on bi-plane data.

For further details on these segmentation methods, reference is made to: Kosaraju A, Makaryus AN. Left Ventricular Ejection Fraction. In: StatPearls [Internet]. Treasure Island (FL): StatPearls Publishing; 2019 Jan-. Available from: https://www.ncbi.nlm.nih.gov/books/NBK459131/.

By way of summary illustration of at least one set of embodiments of the invention, one advantageous system and method in accordance with the invention may be summarized as follows.

The proposed system and method encompasses bi-directional communication between one or more ultrasound imaging apparatuses and a datastore (optionally comprised by a patient monitor) to enable replication of an ultrasound image view that yielded an initial high quality image data set acquired by an expert clinician. This thus enables other clinical staff (who may be unskilled or less skilled in ultrasound image acquisition) to acquire follow-up images over time, which match or correspond to the initial image view, to thereby better monitor the patient. The proposed solution can optionally leverage the patient monitor for data storage, and facilitates the potential use of multiple ultrasound apparatuses during the patient's stay in the medical facility.

The workflow and dataflow might for instance be as follows. When an expert acquires a satisfactory image (e.g. using a TTE or TEE imaging probe, or other non-cardiac imaging probes), the image, along with a time stamp, patient identifier information, optionally operator name, and optionally one or more measurements generated from the image are transferred to and stored in a database on a datastore of the system. The datastore may be comprised by a patient monitor. The datastore may be comprised by an auxiliary device such as a storage module, or simply a personal computer.

When another image and/or measurement is desired at a later point in time, the image corresponding to the previous measurement (and optionally, even earlier measurements) is retrieved from the datastore and loaded on a screen of a user interface of an ultrasound imaging apparatus being used to acquire the new image data, and is used as a reference for the user. The system computes a similarity measure between the live ultrasound image being acquired and the reference image and alerts the user when a match is obtained.

In some embodiments, the similarity measure computation or a separate processing operation may detect anatomical features in the live image data and generate annotation or labelling. For example, one or more frames of the live image data could be labelled according to the anatomical features present in the FOV, or a view depicted by the image could be identified based on the detected anatomical features, and metadata generated for the image view giving a description or labelling of the depicted image view (e.g., LV 4-chamber view label with 4 chambers and valves identified). The system may compare the detected image view with a target (reference) image view for acquisition, and if the second user is acquiring images at an incorrect location, with the incorrect anatomical context (e.g., orthogonal to the desired plane), they may be alerted.

An aspect of the invention also provides a processing arrangement, comprising an input/output and one or more processors. The one or more processors are adapted to perform the following steps:
retrieve at the input/output from a datastore, reference ultrasound image data depicting at least one view of an anatomy of a subject based on querying the datastore with patient identifier information;
receive at the input/output live ultrasound imaging data;
recurrently generate a live similarity measure indicative of similarity between the view represented in the reference image data and a view represented in the live ultrasound image data;
generate a user output alert when the similarity measure matches at least one pre-defined criterion; and
capture and storing one or more frames of the live image data, tagged with the patient identifier information.

This processing arrangement may be comprised by an ultrasound imaging apparatus for example, i.e. the ultrasound imaging device which is acquiring the aforementioned ultrasound imaging data, e.g. the second ultrasound imaging device 22 referred to previously with reference to Fig.1 and Fig. 2. This ultrasound imaging device may form a node in a distributed computing system which also comprises the datastore to which the processing arrangement connects. An ultrasound imaging apparatus comprising the processing arrangement and further comprising an ultrasound imaging probe for acquiring the live ultrasound imaging data forms another aspect of the invention.

The processing arrangement may in general comprise a single processor or a plurality of processors. It may be located in a single containing device, structure or unit, or it may be distributed between a plurality of different devices, structures or units. Reference therefore to the processing arrangement being adapted or configured to perform a particular step or task may correspond to that step or task being performed by any one or more of a plurality of processing components, either alone or in combination. The skilled person will understand how such a distributed processing arrangement can be implemented. The processing arrangement includes a communication module or input/output for receiving data and outputting data to further components.

The one or more processors of the processing arrangement can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

In further examples, the processing arrangement could be a separate processing unit, for example a unit connected as a bridge or interface between a patient monitoring device and an ultrasound imaging device, or more generally as a separate processing module which forms a component of the system and is communicatively connected, linked or networked with the other devices or components of the system.

Another aspect of the invention provides an ultrasound imaging apparatus which comprises the aforementioned processing arrangement, and an ultrasound imaging probe for acquiring the live ultrasound imaging data.

Another aspect of the invention provides a system (e.g. a distributed computing system), in accordance with any of the examples or embodiments outlined above. The system described in association with Fig. 1 for example can be provided as an embodiment of the invention. Also, the system described in association with Fig. 2, including any of the described optional features, examples and variations, can also be provided as an embodiment of the invention.

In one simple example case, an aspect of the invention can provide a system which comprises and ultrasound imaging device or apparatus which includes a processing arrangement as described above, or in accordance with any of the claims of this application; and a datastore storing reference ultrasound image data.

Another aspect of the invention also provides a computer program product comprising code means configured for execution by a processor or a plurality of processors. It is for execution by one or more processors communicatively linked with an ultrasound imaging apparatus and communicatively linked with a datastore.

The code is adapted to cause the processor to perform the following steps:
receive from the datastore, reference ultrasound image data depicting at least one view of an anatomy of a subject based on querying the datastore with patient identifier information;
receive from the ultrasound imaging apparatus live ultrasound image data;
recurrently generate a live similarity measure indicative of similarity between the view represented in the reference image data and a view represented in the live ultrasound image data;
generate a user output alert when the similarity measure matches at least one pre-defined criterion; and
capture and store one or more frames of the live image data, tagged with the patient identifier information.

The code may further cause the processor to output the captured frames of tagged image data to the datastore for storage.

The user alert may be generated by controlling a user interface connected with the processor. The user interface may be a user interface of the ultrasound imaging device, or a separate user interface.

The code may in some examples be run on a processor comprised by the ultrasound imaging device which acquired the ultrasound imaging data. For example, with reference to the embodiment of Fig. 2, this code might be executed by a processor of the second ultrasound imaging apparatus 22.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A computer implemented method, for performance with a distributed computing system, the method comprising:
acquiring at a first ultrasound imaging device (14) first ultrasound imaging data (16) of a subject, including at least one image depicting at least one view of an anatomy of the subject;
storing the first ultrasound imaging data as reference ultrasound image data (20) in a datastore (18), tagged with subject identifier information;
retrieving from the datastore (18), at a second ultrasound imaging device (22), the reference ultrasound image data based on querying the datastore with patient identifier information, wherein the second imaging device is the same as or different to the first imaging device,
acquiring live ultrasound image data at the second ultrasound imaging device;
recurrently generating a live similarity measure indicative of a similarity between the view represented in the reference image data and a view represented in the live ultrasound image data;
generating a user output alert when the similarity measure matches at least one pre-defined criterion,
capturing and storing one or more frames of the live ultrasound image data, tagged with the patient identifier information.

2. The method of claim 1, wherein the pre-defined criterion includes a pre-defined threshold for the similarity measure.

3. The method of claim 1 or 2, wherein the reference image data is retrieved from a datastore comprised by a patient monitoring subsystem.

4. The method of any of claims 1-3, the method comprising capturing and storing at least one frame of the live image data only when the similarity measure meets said pre-defined criterion.

5. The method of claim 4, the method comprising capturing and storing said at least one frame of the live image data
only when the similarity measure meets said pre-defined criterion; and
responsive to a capture command from a user input device.

6. The method of any of claims 1-5, wherein the datastore stores a set of reference images depicting different respective views of the anatomy, each tagged in accordance with the view depicted.

7. The method of claim 6, wherein the datastore further stores a view recommendation indicative of a recommended next view of the anatomy to capture, and wherein the retrieved reference image is one of the set of images depicting the recommended next view.

8. The method of any of claims 1-7, further comprising generating probe positioning guidance based on the similarity metric, the guidance for guiding movement of the probe by a user for improving the similarity metric, and outputting the guidance information to a user interface device.

9. The method of any of claims 1-8, further comprising receiving annotation data for storage in the datastore with the reference image data.

10. The method of any of claims 1-9, wherein the second imaging device is different to the first.

11. The method of any of claims 1-10, further comprising processing the captured at least one frame of the live image data with an anatomical measurement algorithm.

12. A processing arrangement comprising:
an input/output; and
one or more processors, adapted to:
receive at the input/output from a datastore, reference ultrasound image data depicting at least one view of an anatomy of a subject based on querying the datastore with patient identifier information;
receive at the input/output live ultrasound image data;
recurrently generate a live similarity measure indicative of similarity between the view represented in the reference image data and a view represented in the live ultrasound image data;
generate a user output alert when the similarity measure matches at least one pre-defined criterion; and
capture and store one or more frames of the live image data, tagged with the patient identifier information.

13. An ultrasound imaging apparatus comprising:
the processing arrangement of claim 12; and
an ultrasound imaging probe for acquiring the live ultrasound imaging data.

14. A system, comprising:
an ultrasound imaging apparatus as claimed in claim 13; and
a datastore storing reference ultrasound image data.

15. A computer program product comprising code means configured for execution by a processor, wherein, for a processor that is
communicatively linked with an ultrasound imaging apparatus; and
communicatively linked with a datastore,
the code causes the processor to:
receive from the datastore, reference ultrasound image data depicting at least one view of an anatomy of a subject based on querying the datastore with patient identifier information;
receive from the ultrasound imaging apparatus live ultrasound image data;
recurrently generate a live similarity measure indicative of similarity between the view represented in the reference image data and a view represented in the live ultrasound image data;
generate a user output alert when the similarity measure matches at least one pre-defined criterion; and
capture and store one or more frames of the live image data, tagged with the patient identifier information.
